(19) Europäisches Patentamt
European Patent Office
Office européen des brevets

(11) Numéro de publication : **0 483 010 A1**

# (12) DEMANDE DE BREVET EUROPEEN

(21) Numéro de dépôt : **91402847.7**

(22) Date de dépôt : **24.10.91**

(51) Int. Cl.[5] : **C07C 47/42,** C07C 47/45, C07C 45/58, C07D 303/04

(30) Priorité : **25.10.90 FR 9013249**

(43) Date de publication de la demande : **29.04.92 Bulletin 92/18**

(84) Etats contractants désignés : **AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy les Lyon (FR)**
Inventeur : **Delmond, Bernard**
**12 allée des Bergeronnettes**
**F-33600 Pessac (FR)**
Inventeur : **Filliatre, Claude**
**32 rue Georges Bizet**
**F-33400 Talence (FR)**
Inventeur : **Pereyre, Michel**
**45 rue du Haut Carré**
**F-33400 Talence (FR)**
Inventeur : **Serramedan, Dominique**
**37 rue Réaumur**
**F-17000 La Rochelle (FR)**

(74) Mandataire : **Savina, Jacques et al**
**RHONE-POULENC RORER S.A., Direction des Brevets, 90 avenue Raymond Aron**
**F-92165 Antony Cédex (FR)**

## (54) Procédé de préparation du cyclocitral.

(57) L'invention concerne un procédé de préparation du cyclocitral par époxydation du pyronène.

EP 0 483 010 A1

La présente invention concerne un nouveau procédé de préparation du cyclocitral. Plus particulièrement, elle concerne un procédé de préparation du cyclocitral à partir du pyronène.

Le cyclocitral obtenu constitue un intermédiaire de synthèse de la vitamine A et peut donc être utilisé à ce titre.

Le procédé de l'invention est caractérisé en ce que l'on effectue les étapes suivantes:

– dans une première étape, on époxyde le pyronène de formule (1)

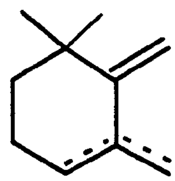

(1)

dans laquelle la ligne discontinue représente une seule double liaison qui peut ainsi être située en position $\gamma$ ou $\delta$, et,

– dans une deuxième étape, on traite le produit issu de la première étape pour former le cyclocitral de formule (2) :

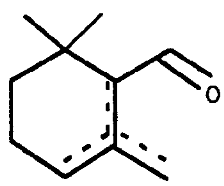

(2)

dans laquelle la ligne discontinue a la même signification que précédemment, définissant le cyclocitral $\alpha$, $\beta$ ou $\gamma$.

Les produits de départ sont ainsi le triméthyl-1,5,5 méthylène-6 cyclohexène-1 (ou encore, le gamma pyronène); et le Diméthyl-1,1 diméthylène-2,3 cyclohéxane (ou encore, le $\delta^3$-pyronène).

Avantageusement, le pyronène de départ est le $\delta^3$-pyronène de formule :

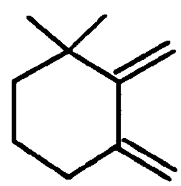

On connait dans l'art antérieur un moyen de préparer le cyclocitral à partir d'une cétone insaturée, par formation de l'époxyde intermédiaire au moyen d'un ylure de soufre (Rosenberger et Coll., Helvetica Chimica Acta Vol. 63 Fasc. 6 (1980).

Cependant, la cétone de départ (triméthylcyclohexenone) est un produit coûteux, difficilement accessible, ce qui ne permet pas l'exploitation industrielle de ce procédé.

Le cyclocitral peut également être préparé par cyclisation du citralanil et hydrolyse du groupement amine protecteur (Henbest et coll., J. Chem. Soc. 1154 (1952)). Toutefois, ce mécanisme implique des étapes multiples et l'utilisation d'un produit toxique : l'aniline.

Rien dans ces documents ne décrit ni ne suggère la possibilité de préparer le cyclocitral à partir du pyronène. Le procédé de l'invention constitue un nouveau moyen d'accès au cyclocitral particulièrement efficace, permettant de valoriser des matières premières de départ peu coûteuses et aisément accessibles.

Plus particulièrement, la première étape du procédé est réalisée au moyen d'un agent époxydant qui peut être choisi parmi les peracides ou leurs dérivés, l'eau oxygénée, les hydroperoxydes d'alkyles, les perborates ou les percarbonates.

Parmi les peracides organiques utilisables dans la présente invention, on peut citer les acides et les dérivés d'acides carboxyliques, aliphatiques ou aromatiques, éventuellement substitués. Notamment, l'acide peracétique, l'acide performique, l'acide perpropionique, l'acide pertrifluoroacétique, l'acide paranitroperbenzoïque, ou l'acide métachloroperbenzoïque peuvent être employés.

De plus, dans un mode particulier de mise en oeuvre du procédé de l'invention, il est possible de synthétiser

directement "in situ" le peracide en utilisant un mélange d'eau oxygénée et d'acide correspondant. Dans ce cas, l'acide peut être utilisé en quantités catalytiques puisque, au cours de la réaction du peracide sur le pyronène, l'acide de départ est régénéré et peut être recyclé en peracide en réagissant avec l'eau oxygénée.

En ce qui concerne l'eau oxygénée, elle peut être utilisée seule, en milieu basique, ou combinée:

- à un métal, ou
- à un nitrile (réaction de Radziszewski: Wiberg, J.Amer.Chem.Soc., 75, 3961 (1953)).

Parmi les métaux qui conviennent dans le procédé lorsque l'on utilise l'eau oxygénée, on peut citer les métaux de transition tels que notamment le tungstène, le molybdène, le vanadium, le titane, le platine ou le manganèse, éventuellement associés à un autre métal, tel que notamment l'étain.

Préférentiellement, on utilise le tungstène, le molybdène ou le platine.

Dans le cas des hydroperoxydes, on utilise comme agent époxydant l'association ROOH + Métal dans laquelle R est un radical alkyl et le métal est choisi parmi les métaux de transition tel le vanadium, le titane, le molybdène, le platine ou le cobalt.

Préférentiellement, l'hydroperoxyde a pour formule :

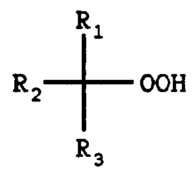

dans laquelle R1 à R3, identiques ou différents, représentent :

- des atomes d'hydrogène,
- des groupements alkyles linéaires ou ramifiés qui comportent de 1 à 30 atomes de carbone,
- des groupements cycloalkyles qui comportent de 3 à 12 atomes de carbone, ou
- des groupements alkyl- ou cycloalkylaromatiques qui comportent de 7 à 30 atomes de carbone.

Parmi les métaux utilisables lorsque l'agent époxydant est un hydroperoxyde d'alkyle, on préfère le vanadium et le titane.

La réaction d'époxydation peut également être réalisée en présence de perborate ou de percarbonate. Notamment, parmi les percarbonates et les perborates utilisables dans la présente invention, on peut citer le percarbonate de sodium (Chem Letters 665 (1986)), le perborate de sodium (Tetrahedron Letters, 2967 (1988)), et les perborates d'alkyles (FR 1,447,267), dont l'effet sur l'époxydation des alcènes a été décrit.

La réaction d'époxydation est effectuée dans un milieu inerte en présence d'un solvant choisi parmi:

- l'eau;
- les solvants étherés tels que l'éther éthylique ou propylique, le THF ou encore le méthylterbutyléther;
- les solvants halogénés tels que notamment les chlorobenzènes, le chloroforme, le chlorure de méthylène ou le dichloropropane;
- les hydrocarbures aliphatiques ou aromatiques, et notamment les alcanes ayant plus de 5 atomes de carbone (hexane, heptane);
- les acides organiques tels que l'acide acétique ou l'acide formique;
- les alcools ou
- les esters.

Les différents réactifs peuvent être introduits simultanément mais on préfère ajouter l'agent époxydant sur le pyronène dissout dans le solvant.

Par ailleurs, il est possible d'ajouter au milieu un agent de transfert de phase pour faire de la catalyse par transfert de phase. Notamment, on peut ajouter :

- des sels d'ammonium quaternaire tels que l'hydroxyde, le bromure ou le chlorure de tétrabutylammonium, le chlorure de methyltrioctylammonium, ou le chlorure de diméthyl[dioctadécyl + dihexadécyl]ammonium;
- des hydrocarbures aromatiques ou chlorés;
- des sels de phosphonium, tels que notamment le chlorure d'hexadecyltributylphosphonium;
- certains complexes anioniques comme le tetrahexylammonium tetra(diperoxotungsto)phosphate.

La température de la réaction est de préférence comprise entre -30°C et +100°C, et encore plus préférentiellement entre 0°C et 50°C. Il peut être particulièrement avantageux d'opérer à une température proche de la température ambiante.

Le rapport molaire agent époxydant/pyronène est avantageusement compris entre 0,5 et 1,5 et de préférence proche de 1.

Les conditions réactionnelles (température, nature et quantité du solvant et de l'agent époxydant, durée de la réaction) sont adaptées par l'homme de l'art à la vitesse optimale de réaction désirée et à la nature des isomères recherchés.

Au cours de la première étape du procédé, la formation des produits intermédiaires de formule (3) a été mise en évidence, dans laquelle la ligne discontinue représente une seule double liaison :

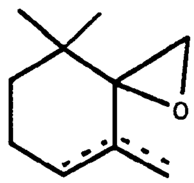

(3)

Il s'agit ainsi du Diméthyl-4,4 méthylène-8 oxa-1 spiro(2,5) octane et du Triméthyl-4,8,8 oxa-1 spiro (2,5) octène-4, qui peuvent être purifiés.

Par ailleurs, lors de la mise en oeuvre de la première étape du procédé de l'invention, il peut se former des produits secondaires d'époxydation. Notamment les produits suivants ont été isolés :

(4) 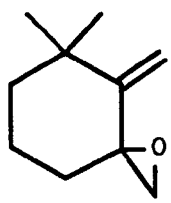 et 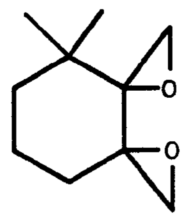 (5)

Ils peuvent être éventuellement séparés des produits de formule (3) avant la mise en oeuvre de la seconde étape du procédé, notamment par chromatographie en phase liquide. Mais la seconde étape du procédé peut aussi être réalisée directement sur le mélange. Dans ce cas, le cyclocitral de formule (2) est isolé du produit terminal par toute technique connue de l'homme de l'art.

La seconde étape du procédé consiste à traiter le produit issu de la première étape pour former le cyclocitral de formule (2).

En fonction du produit de départ et des conditions réactionnelles, il est possible de former préférentiellement les isomères $\alpha$, $\beta$ ou $\gamma$ du cyclocitral correspondant aux 3 positions possibles de la double liaison.

Cette étape est conduite en présence d'un solvant qui peut être choisi parmi les solvants étherés tels que l'éther éthylique ou propylique, le THF ou encore le méthylterbutyléther; les solvants halogénés tels que notamment les chlorobenzènes, le chloroforme, le chlorure de méthylène ou le dichloropropane; les alcanes, et notamment les alcanes ayant plus de 5 atomes de carbone (hexane, heptane); les acides organiques tels que l'acide acétique, l'acide formique ou l'acide p-toluène sulfonique; les alcools; les solvants aromatiques ou les esters. Il peut être particulièrement avantageux d'utiliser le même solvant que lors de la première étape.

Par ailleurs, la réaction est effectuée de préférence en atmosphère inerte.

Différents types de traitement peuvent être utilisés pour former le cyclocitral (2). Notamment, la réaction peut être effectuée par chauffage, ou au moyen d'un catalyseur, et en particulier d'un catalyseur métallique ou acide.

Différents types de catalyseurs acides peuvent être utilisés, tels que notamment des acides protoniques (l'acide p-toluène sulfonique), des acides de Lewis (bromure de zinc, bromure de magnésium, alkylaluminates, trifluorure de bore, chlorure d'aluminium, chlorure de zinc, chlorure de titane, chlorure d'étain) ou des oxydes (alumine).

Concernant les catalyseurs métalliques, on peut utiliser notamment MgBr2, LiBF4, BF3.OEt2, ou différents métaux de transition tels qu'en particulier les complexes du palladium, le molybdène ou le rhodium. Il est aussi possible d'utiliser d'autres métaux tels que notamment le cuivre.

Enfin, ces différents types de catalyseurs métalliques peuvent être utilisés sur un support, comme par exemple le graphite.

La température de la réaction est adaptée au catalyseur utilisé. Préférentiellement, lorsque cela est possible, la température est identique à celle de la première étape du procédé.

La seconde étape du procédé peut être enchainée directement sur la première dans la même enceinte réactionnelle ou réalisée après séparation de l'époxyde formé.

Le pyronène de départ utilisé dans la présente invention peut être obtenu de différentes façons. En particulier, il peut être préparé à partir du myrcène selon le protocole suivant (demande de brevet française FR 9002724) :

– mise en contact du myrcène avec le dioxyde de soufre en présence d'un inhibiteur de polymérisation, à une température comprise entre 60 et 100°C, pour former la myrcène sulfone,

– traitement de la myrcène sulfone en présence d'un acide fort contenant moins de 5 % d'eau pour former le sulfolène cyclique, et

– chauffage du sulfolène cyclique, éventuellement en présence d'un catalyseur basique pour former le pyronène.

Au cours de la seconde étape, on peut utiliser comme acide fort les acides alkyl, aryl ou halogénosulfoniques, les résines Nafions, l'acide perchlorique, l'acide sulfurique ou différents catalyseurs hétérogènes acides.

Le cyclocitral de formule (2) obtenu selon l'invention peut être utilisé comme intermédiaire de synthèse de la vitamine A (Chem.& Ind. p574 (1950); Chem. Letters p 1201 (1975). Il peut également être utilisé pour préparer le safranal (Tetrahedron Letters 36, p 3175 (1974)).

L'invention sera plus complètement décrite à l'aide des exemples qui suivent, qui doivent être considérés comme illustratifs et non limitatifs.

EXEMPLE 1 SYNTHESE DE L'EPOXYDE DU $\delta^3$ PYRONENE

Dans un grignard de 250 ml, on dilue 2,5 g de pyronène dans 100 ml d'éther anhydre, sous $N_2$ et à 0°C. Puis, on additionne 4 g d'acide métachloroperbenzoïque (80-90 %). On laisse remonter à température ambiante et on maintient l'agitation pendant 48 heures.

On dilue avec 50 ml d'éther, on lave avec une solution 10 % de bisulfite de sodium (2 x 50 ml), puis avec une solution saturée de bicarbonate de sodium (4 x 50 ml). On sèche sur $MgSO_4$, on évapore le solvant. S'il subsiste de l'acide, on reprend avec une petite quantité de pentane et on filtre. On purifie sur $Al_2O_3$ (6 % $H_2O$) : éluant éther de pétrole ou pentane/éther, 9/1.

On obtient 1,7 g d'époxyde (Rdt = 60 %).

EXEMPLE 2

Dans un ballon de 50 ml, muni d'une forte agitation et placé sous $N_2$, on introduit 25 g d'$Al_2O_3$ active grade I (MERCK), 30 ml d'éther anhydre et 1 g d'époxyde du $\delta$ $^3$-pyronène préparé comme dans l'exemple 1. D'autre part, on se place à l'abri de la lumière et on prend la précaution d'ajouter une pincée d'hydroquinone. On laisse agiter 15 heures à température ambiante. On ajoute 20 ml d'éther et on agite encore 15 minutes.

On filtre, on rince le filtrat avec 100 ml d'éther, 100 ml de $CHCl_3$, 100 ml d'un mélange 1:1, éther: méthanol. On évapore les solvants.

On obtient avec un rendement de 94 % un produit brut titrant en CPV :

| | |
|---|---|
| - $\alpha$ cyclocitral | 4 % |
| - $\beta$ cyclocitral | 84 % |
| - $\gamma$ cyclocitral | 12 % |

EXEMPLE 3

Dans un grignard de 100 ml, muni d'un réfrigérant, d'une agitation magnétique et placé sous $N_2$, on introduit 0,5 ml de $Br_2$, à 10°C sur 750 mg de Mg en copeaux, dans 35 ml d'éther anhydre.

Après une demi-heure, on chauffe 5 heures au reflux, puis on laisse la nuit à une température ambiante et sous $N_2$. On additionne ensuite 500 mg d'époxyde de $\delta^3$-pyronène dans 15 ml de benzène anhydre. On chauffe 1 heure au reflux. On laisse revenir à température ambiante et on hydrolyse sur glace et lave avec une solution saturée de $NH_4Cl$ jusqu'à neutralité. On sèche sur $MgSO_4$, on évapore les solvants. On obtient avec un rendement de 98 % un produit brut titrant en CPV:

- $\gamma$ cyclocitral 88 %

EXEMPLE 4

Dans un ballon de 100 ml, on place 890 mg (3,95 mmole) de bromure de zinc fondu dans 50 ml de benzène et sous $N_2$.

On introduit 500 mg (3,29 mmole) d'époxyde du $\delta$-pyronène, dissout dans quelques millilitres de benzène. On porte 45 minutes au reflux, puis on laisse revenir à température ambiante. On hydrolyse sur glace et lave

avec une solution saturée de $NH_4Cl$ jusqu'à neutralité, puis on extrait les phases aqueuses avec 2 x 50 ml d'éther. On sèche sur $MgSO_4$ et on évapore les solvants.

On obtient 420 mg de produit brut.

Après purification sur colonne $Al_2O_3$ désactivée (6 % $H_2O$) on obtient avec un rendement de 84% :

| | |
|---|---|
| - α cyclocitral majoritaire | 86 % |
| - γ cyclocitral | 14 % |

EXEMPLE 5

Dans un ballon de 30 ml, on place 300 mg (1, 97 mmoles) d'époxyde du δ-pyronène dans 15 ml de benzène anhydre. On ajoute ensuite 94 mg (0, 4 mmoles) d'acide p-toluène sulfonique.

On porte 3 heures au reflux, puis laisse revenir à température ambiante. On hydrolyse sur de la glace, extrait à l'éther, lave avec une solution saturée de bicarbonate de sodium et sèche sur sulfate de magnésium. Après évaporation des solvants, on obtient 285 mg d'un mélange d'isomères ß et γ que l'on dose en CPV capillaire:

| | |
|---|---|
| - β cyclocitral | 90 % |
| - γ cyclocitral | 10 % |

Rendement = 95 %.

## Revendications

**1 -** Procédé de préparation du cyclocitral caractérisé en ce que l'on effectue les étapes suivantes :

. dans une première étape, on époxyde le pyronène de formule (1) :

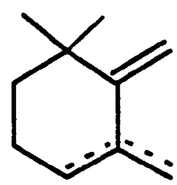

(1)

dans laquelle la ligne discontinue représente une seule double liaison, et,

. dans une deuxième étape, on traite le produit issu de la première étape pour former le cyclocitral de formule (2) :

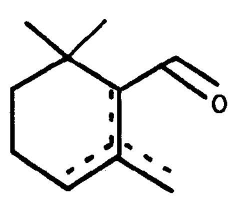

(2)

dans laquelle la ligne discontinue a la même signification que précédemment.

**2 -** Procédé selon la revendication 1 caractérisé en ce que le pyronène de départ est le $\delta^3$-pyronène de formule :

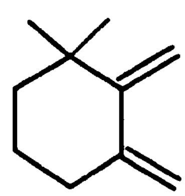

3 - Procédé selon les revendications 1 et 2 caractérisé en ce que l'époxydation est réalisée au moyen d'un agent époxydant choisi parmi les peracides et leurs dérivés, l'eau oxygénée, les hydroperoxydes d'alkyles, les perborates et les percarbonates.

**4** - Procédé selon la revendication 3 caractérisé en ce que le peracide est un acide ou un dérivé d'acide carboxylique aliphatique ou aromatique éventuellement substitué.

**5** - Procédé selon la revendication 4 caractérisé en ce que le peracide est choisi parmi les acides peracétique, performique, perpropionique, pertrifluoroacétique, paranitroperbenzoïque, et métachloroperbenzoïque.

**6** - Procédé selon l'une quelconque des revendications 3 à 5 caractérisé en ce que le peracide est formé "in situ".

**7** - Procédé selon la revendication 3 caractérisé en ce que l'eau oxygénée est utilisée seule, en milieu basique, ou combinée à un nitrile ou à un métal, et de préférence à un métal de transition éventuellement associé à un autre métal.

**8** - Procédé selon la revendication 3 caractérisé en ce que l'hydroperoxyde d'alkyle consiste en la combinaison ROOH/métal dans laquelle R est un radical alkyl et le métal est un métal de transition.

**9** - Procédé selon la revendication 8 caractérisé en ce que l'hydroperoxyde a pour formule.

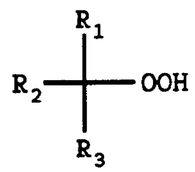

dans laquelle R1 à R3, identiques ou différents, représentent :

– des atomes d'hydrogène,
– des groupements alkyles linéaires ou ramifiés qui comportent de 1 à 30 atomes de carbone,
– des groupements cycloalkyles qui comportent de 3 à 12 atomes de carbone, ou
– des groupements alkyl- ou cycloalkylaromatiques gui comportent de 7 à 30 atomes de carbone.

**10** - Procédé selon l'une quelconque des revendications 7 à 9 caractérisé en ce que le métal de transition est choisi parmi le tungstène, le molybdène, le vanadium, le titane, le platine, le manganèse, et le cobalt.

**11** - Procédé selon la revendication 10 caractérisé en ce que lorsque l'agent époxydant est l'eau oxygénée, le métal est choisi parmi le tungstène, le molybdène et le platine.

**12** - Procédé selon la revendication 10 caractérisé en ce que lorsque l'agent époxydant est un hydroperoxyde d'alkyle, le métal est choisi parmi le vanadium et le titane.

**13** - Procédé selon la revendication 3 caractérisé en ce que la réaction est effectuée en atmosphère inerte, à une température comprise entre -30°C et +100°C, et de préférence entre 0°C et 50°C.

**14** - Procédé selon la revendication 3 caractérisé en ce que le rapport molaire agent époxydant/pyronène est compris entre 0,5 et 1,5, et de préférence proche de 1.

**15** - Procédé selon la revendication 1 caractérisé en ce que, dans la deuxième étape, on traite le produit issu de la première étape par la chaleur, et/ou en présence d'un catalyseur acide ou métallique.

**16** - Procédé selon la revendications 15 caractérisé en ce que le catalyseur acide est choisi parmi les acides protoniques, les acides de Lewis et les oxydes.

**17** - Procédé selon la revendications 15 caractérisé en ce que le catalyseur métallique est supporté.

Office européen des brevets

# RAPPORT DE RECHERCHE EUROPEENNE

Numero de la demande

EP 91 40 2847

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | BULLETIN DE LA SOCIETE CHIMIQUE DE FRANCE no. 5-6, 1974, PARIS FR pages 963 - 968; A. SEVIN ET AL.: 'No 188. - Cinétique et Stéréochimie d'Epoxydation de Systèmes Méthylènecyclohexaniques.' * Pages 963, 967 * | 1,3-5, 13,14 | C07C47/42 C07C47/45 C07C45/58 C07D303/04 |
| A | HELVETICA CHIMICA ACTA vol. 57, no. 121, 1974, BASEL CH pages 1098 - 1116; J. EHRENFREUND ET AL.: 'Oxydation von Allylalkoholen mit Blei(IV)-Acetat.' * Pages 1099, 1105-1106 * | 1 | |
| A | CH-A-642 962 (FIRMENICH S.A.) * Revendication 1; page 4; page 5, point b * | 1,15,16 | |
| A | EP-A-0 374 509 (FIRMENICH S.A.) * page 3 - page 5; revendications; exemples 1,5 * | 1,3-5, 15,16 | |
| | | | **DOMAINES TECHNIQUES RECHERCHES (Int. Cl.5 )** |
| | | | C07C |

Le présent rapport a été établi pour toutes les revendications

| Lieu de la recherche | Date d'achèvement de la recherche | Examinateur |
|---|---|---|
| LA HAYE | 23 JANVIER 1992 | BONNEVALLE E.I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)